# EUROPÄISCHE PATENTSCHRIFT

(11) **EP 1 493 034 B1**
(45) Veröffentlichungstag und Bekanntmachung des Hinweises auf die Patenterteilung: **05.01.2011**
(21) Anmeldenummer: 03722485.4
(22) Anmeldetag: 15.04.2003
(51) Int. Cl.: G01N 33/68, G01N 33/574, C07K 14/47, A61K 38/17

(54) **VERFAHREN ZUR DIAGNOSE VON ENTZÜNDUNGSERKRANKUNGEN UND INFEKTIONEN MITTELS BESTIMMUNG VON LASP-1/ LAP-1**
METHOD FOR DIAGNOSING INFLAMMATORY DISEASES AND INFECTIONS BY THE DETERMINATION OF LASP-1 IMMUNOREACTIVITY
METHODE POUR DIAGNOSTIQUER DES MALADIES INFLAMMATOIRES ET DES INFECTIONS PAR DETERMINATION DE LASP-1-IMMUNOREACTIVITE

(30) Priorität: 19.04.2002 EP 02008840
(43) Veröffentlichungstag der Anmeldung: 05.01.2005
(73) Patentinhaber: B.R.A.H.M.S GmbH, 16761 Hennigsdorf (DE)
(72) Erfinder: BERGMANN, Andreas, 12351 Berlin (DE); FISCHER-SCHULZ, Christina, 13507 Berlin (DE); STRUCK, Joachim, 12161 Berlin (DE)
(74) Vertreter: Andrae, Steffen
(86) Internationale Anmeldenummer: PCT/EP2003/003940
(87) Internationale Veröffentlichungsnummer: WO 2003/089934

(56) Entgegenhaltungen:
- WO-A-00/22439
- WO-A-01/25268
- WO-A-01/36975
- WO-A-01/85986
- WO-A-01/86288
- WO-A-02/00857
- WO-A-02/10771
- WO-A-92/10200
- WO-A-98/27213
- DE-A- 19 915 485
- US-A- 5 981 218
- SCHREIBER VALERIE ET AL: "Lasp-1, a novel type of actin-binding protein accumulating in cell membrane extensions." MOLECULAR MEDICINE (NEW YORK), Bd. 4, Nr. 10, Oktober 1998 (1998-10), Seiten 675-687, XP008019832 ISSN: 1076-1551
- BUTT ELKE ET AL: "Actin binding of human LIM and SH3 protein is regulated by cGMP- and cAMP-dependent protein kinase phosphorylation on serine 146." JOURNAL OF BIOLOGICAL CHEMISTRY, Bd. 278, Nr. 18, 2. Mai 2003 (2003-05-02), Seiten 15601-15607, XP002248493 ISSN: 0021-9258
- LICASTRO F ET AL: "Increased plasma levels of interleukin-1, interleukin-6 and alpha-1-antichymotrypsin in patients with Alzheimer's disease: Peripheral inflammation or signals from the brain?" JOURNAL OF NEUROIMMUNOLOGY, vol. 103, no. 1, 1 February 2000 (2000-02-01), pages 97-102,
- LICASTRO F ET AL: "Increased plasma levels of interleukin-1, interleukin-6 and alpha-1-antichymotrypsin in patients with Alzheimer's disease: Peripheral inflammation or signals from the brain?" JOURNAL OF NEUROIMMUNOLOGY, vol. 103, no. 1, 1 February 2000 (2000-02-01), pages 97-102,

### SEQUENCE LISTING

<110> B.R.A.H.M.S Aktiengesellschaft
<120> Verfahren zur Diagnose von Entzündungserkrankungen und Infektionen unter Bestimmung von LASP-1 Immunreaktivität
<130> 3677PCT AS
<140>
   <141>
<150> 02008840.7
   <151> 2002-04-19
<160> 17
<170> PatentIn Ver. 2.1
<210> 1
   <211> 261
   <212> PRT
   <213> Homo sapiens
<400> 1
   Met Asn Pro Asn Cys Ala Arg Cys Gly Lys Ile Val Tyr Pro Thr Glu
    1               5                  10                  15
   Lys Val Asn Cys Leu Asp Lys Phe Trp His Lys Ala Cys Phe His Cys
               20                  25                  30
   Glu Thr Cys Lys Met Thr Leu Asn Met Lys Asn Tyr Lys Gly Tyr Glu
           35                  40                  45
   Lys Lys Pro Tyr Cys Asn Ala His Tyr Pro Lys Gln Ser Phe Thr Met
       50                  55                  60
   Val Ala Asp Thr Pro Glu Asn Leu Arg Leu Lys Gln Gln Ser Glu Leu
   65                  70                  75                  80
   Gln Ser Gln Val Arg Tyr Lys Glu Glu Phe Glu Lys Asn Lys Gly Lys
                   85                  90                  95
   Gly Phe Ser Val Val Ala Asp Thr Pro Glu Leu Gln Arg Ile Lys Lys
               100                 105                 110
   Thr Gln Asp Gln Ile Ser Asn Ile Lys Tyr His Glu Glu Phe Glu Lys
           115                 120                 125
   Ser Arg Met Gly Pro Ser Gly Gly Glu Gly Met Glu Pro Glu Arg Arg
       130                 135                 140
   Asp Ser Gln Asp Gly Ser Ser Tyr Arg Arg Pro Leu Glu Gln Gln Gln
   145                 150                 155                 160
   Pro His His Ile Pro Thr Ser Ala Pro Val Tyr Gln Gln Pro Gln Gln
                   165                 170                 175
   Gln Pro Val Ala Gln Ser Tyr Gly Gly Tyr Lys Glu Pro Ala Ala Pro
               180                 185                 190
   Val Ser Ile Gln Arg Ser Ala Pro Gly Gly Gly Gly Lys Arg Tyr Arg
           195                 200                 205
   Ala Val Tyr Asp Tyr Ser Ala Ala Asp Glu Asp Glu Val Ser Phe Gln
       210                     215                     220
   Asp Gly Asp Thr Ile Val Asn Val Gln Gln Ile Asp Asp Gly Trp Met
   225                     230                     235                     240
   Tyr Gly Thr Val Glu Arg Thr Gly Asp Thr Gly Met Leu Pro Ala Asn
                   245                     250                     255
   Tyr Val Glu Ala Ile
               260
<210> 2
   <211> 10
   <212> PRT
   <213> Homo sapiens
<400> 2
   Gln Gln Ser Glu Leu Gln Ser Gln Val Arg
   1               5                   10
<210> 3
   <211> 9
   <212> PRT
   <213> Homo sapiens
<400> 3
   Ala Cys Phe His Cys Glu Thr Cys Lys
   1               5
<210> 4
   <211> 10
   <212> PRT
   <213> Homo sapiens
<400> 4
   Lys Pro Tyr Cys Asn Ala His Tyr Pro Lys
   1               5                   10
<210> 5
   <211> 10
   <212> PRT
   <213> Homo sapiens
<400> 5
   Val Asn Cys Leu Asp Lys Phe Trp His Lys
   1               5                   10
<210> 6
   <211> 13
   <212> PRT
   <213> Homo sapiens
<400> 6
   Gly Phe Ser Val Val Ala Asp Thr Pro Glu Leu Gln Arg
   1 5 10
<210> 7
   <211> 12
   <212> PRT
   <213> Homo sapiens
<400> 7
   Leu Lys Gln Gln Ser Glu Leu Gln Ser Gln Val Arg
   1 5 10
<210> 8
   <211> 14
   <212> PRT
   <213> Homo sapiens
<400> 8
   Met Gly Pro Ser Gly Gly Glu Gly Met Glu Pro Glu Arg Arg
   1 5 10
<210> 9
   <211> 19
   <212> PRT
   <213> Artificial Sequence
<220>
   <223> Description of Artificial Sequence: Synthetic Peptide
<400> 9
   Cys Val Tyr Gln Gln Pro Gln Gln Gln Pro Val Ala Gln Ser Tyr Gly
   1 5 10 15
   Gly Tyr Lys
<210> 10
   <211> 15
   <212> PRT
   <213> Homo sapiens
<400> 10
   Thr Gly Asp Thr Gly Met Leu Pro Ala Asn Tyr Val Glu Ala Ile
   1 5 10 15
<210> 11
   <211> 15
   <212> PRT
   <213> Homo sapiens
<400> 11
   Gly Lys Gly Phe Ser Val Val Ala Asp Thr Pro Glu Leu Gln Arg
   1 5 10 15
<210> 12
   <211> 14
   <212> PRT
   <213> Homo sapiens
<400> 12
   Gln Ser Phe Thr Met Val Ala Asp Thr Pro Glu Asn Leu Arg
   1 5 10
<210> 13
   <211> 18
   <212> PRT
   <213> Artificial Sequence
<220>
   <223> Description of Artificial Sequence: Synthetic Peptide
<400> 13
   Cys Lys Tyr His Glu Glu Phe Glu Lys Ser Arg Met Gly Pro Ser Gly
   1 5 10 15
   Gly Glu
<210> 14
   <211> 14
   <212> PRT
   <213> Artificial Sequence
<220>
   <223> Description of Artificial Sequence: Synthetic Peptide
<400> 14
   Cys Gln Asp Gly Ser Ser Tyr Arg Arg Pro Leu Glu Gln Gln
   1 5 10
<210> 15
   <211> 54
   <212> PRT
   <213> Artificial Sequence
<220>
   <223> Description of Artificial Sequence: Synthetic Peptide
<400> 15
   Lys Tyr His Glu Glu Phe Glu Lys Ser Arg Met Gly Pro Ser Gly Gly
    1               5                  10                  15
   Glu Gly Gly Gly Gln Asp Gly Ser Ser Tyr Arg Arg Pro Leu Glu Gln
               20                  25                  30
   Gln Gly Gly Gly Val Tyr Gln Gln Pro Gln Gln Gln Pro Val Ala Gln
           35                  40                  45
   Ser Tyr Gly Gly Tyr Lys
       50
<210> 16
   <211> 323
   <212> PRT
   <213> Homo sapiens
<400> 16
   Met Asn Pro Asn Cys Ala Arg Cys Gly Lys Ile Val Tyr Pro Thr Glu
    1               5                  10                  15
   Lys Val Asn Cys Leu Asp Lys Phe Trp His Lys Ala Cys Phe His Cys
               20                  25                  30
   Glu Thr Cys Lys Met Thr Leu Asn Met Lys Asn Tyr Lys Gly Tyr Glu
           35                  40                  45
   Lys Lys Pro Tyr Cys Asn Ala His Tyr Pro Lys Gln Ser Phe Thr Met
       50                  55                  60
   Val Ala Asp Thr Pro Glu Asn Leu Arg Leu Lys Gln Gln Ser Glu Leu
   65                  70                  75                  80
   Gln Ser Gln Val Arg Tyr Lys Glu Glu Phe Glu Lys Asn Lys Gly Lys
                   85                  90                  95
   Gly Phe Ser Val Val Ala Asp Thr Pro Glu Leu Gln Arg Ile Lys Lys
               100                 105                 110
   Thr Gln Asp Gln Ile Ser Asn Ile Lys Tyr His Glu Glu Phe Glu Lys
           115                 120                 125
   Ser Arg Met Gly Pro Ser Gly Gly Glu Gly Met Glu Pro Glu Arg Arg
       130                 135                 140
   Asp Ser Gln Asp Gly Ser Ser Tyr Arg Arg Pro Leu Glu Gln Gln Gln
   145                 150                 155                 160
   Pro His His Ile Pro Thr Ser Ala Pro Val Tyr Gln Gln Pro Gln Gln
                   165                 170                 175
   Gln Pro Val Ala Gln Ser Tyr Gly Gly Tyr Lys Glu Pro Ala Ala Pro
               180                 185                 190
   Val Ser Ile Gln Arg Ser Ala Pro Ile Cys Leu Gln His Ile Pro Arg
           195                 200                 205
   His Arg Ile Arg Pro Gly Arg Asp Pro Ser Ile Leu Gln Cys Leu Cys
       210                 215                 220
   Phe Leu Lys Pro Ala Thr Ala Cys Asp Ser Tyr Pro Ser Ser Ser Phe
   225                 230                 235                 240
   Phe Cys Gln Leu Lys Pro Ser Ser Ala Thr Ser Ala Gly Ser Leu Leu
                   245                 250                 255
   Trp Gln Ala Ser Pro Leu Ile Asp Phe Leu Val Phe Ser Leu Asp Gly
               260                 265                 270
   Thr Gly Met Gly Leu Ser Gly Gly Gly Arg Gly Pro Trp Gly Arg Ala
           275                 280                 285
   Gly Met Gly Asp Leu Leu Ala Cys Gly Pro His Leu Pro Leu Cys Ser
       290                 295                 300
   Leu Pro Ser His Pro Pro Ala Gln Leu Leu Thr Tyr Pro His Ile Pro
   305                 310                 315                 320
   Gly Leu Gly
<210> 17
   <211> 19
   <212> PRT
   <213> Artificial Sequence
<220>
   <223> Description of Artificial Sequence: Synthetic Peptide
<400> 17
   Cys Val Tyr Gln Gln Pro Gln Gln Gln Pro Val Ala Gln Ser Tyr Gly
     1               5                  10                  15
   Gly Tyr Lys

## Beschreibung

Die vorliegende Erfindung betrifft neue Verwendungen des Proteins LASP-1 sowie ggf. eng verwandter Proteine, die wenigstens hinsichtlich ihrer ersten 200 Aminosäuren die gleiche Sequenz wie LASP-1 und damit eine vergleichbare Immunreaktivität aufweisen, sowie von Fragmenten davon, für die medizinischen Diagnostik von Sepsis und sepsisähnlichen systemischen Infektionen sowie von Herzinfarkten und der Alzheimer Krankheit gemäß Anspruch 1. Sie beruht auf dem erstmaligen Nachweis stark erhöhter Konzentrationen des Proteins LASP-1 im Gehirngewebe von Primaten, bei denen experimentell durch Toxinverabreichunge eine Sepsis bzw. systemische Entzündung hervorgerufen wurde, sowie auf dem anschließenden Nachweis einer stark erhöhten LASP-1 Immunreaktivität in der Zirkulation von Patienten mit Sepsis und von Alzheimer-Patienten, wobei eine erhöhte entsprechende Immunreaktivität auch bei Patienten mit Herzinfarkt festgestellt wurde.

Wenn in der nachfolgenden Beschreibung von LASP-1 gesprochen wird, soll dieser Begriff nicht nur das bekannte Protein LASP-1 mit der in SEQ ID NO:1 angegebenen konkreten Aminosäuresequenz bezeichnen, sondern ggf. auch verwandte Proteine, die innerhalb der ersten 200 Aminosäuren mit diesem LASP-1 sequenzidentisch sind, z.B. das Protein LAP-1, zu dem sich am Ende des experimentellen Teils dieser Anmeldung noch erläuternde Ausführungen finden. Diese Definition gilt für alle Bezugnahmen auf LASP-1, es sei denn, es ergibt sich aus dem konkreten Zusammenhang für den Fachmann etwas anderes. Wenn von LASP-1 gesprochen wird, meint dieser Begriff außerdem auch Fragmente mit einer LASP-1-Immunreaktivität, wobei alle genannten Produkte sowohl frei als auch ggf. an Bindeproteine gebunden und/oder in einer posttranslational modifizierten Form, z.B. in glycosylierter und/oder phosphorylierter Form, vorliegen können.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen. Als Erkrankung, die von typischen Symptomen einer Entzündung, und zwar des Gehirns, begleitet wird, wird im Zusammenhang der vorliegenden Patentanmeldung auch die Alzheimer Krankheit angesehen, die durch eine in der Regel im 5. bis 6. Lebensjahrzehnt auftretende, unaufhaltsam fortschreitende Großhirnrindenatrophie charakterisiert ist und aufgrund ihrer Häufigkeit eine große volkswirtschaftliche Bedeutung aufweist.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α, Interleukin-1) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß oder vermutet, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen oder Ausprägungen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund (gute Spezifität und Selektivität), ohne dass dessen Rolle in der komplexen Kaskade der am Entzündungsgeschehen beteiligten endogenen Substanzen bekannt sein muss.

Eine bekannte, als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Infektionsgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere peptidische Substanzen, die Prohormon-Immunreaktivität aufweisen, signifikant erhöhte Konzentrationen beobachtet werden können. Während das beschriebene Phänomen gut dokumentiert ist, sind die Ursachen für den Anstieg der Prohormon-Immunreaktivität bei Sepsis weiterhin weitgehend ungeklärt.

In der vorliegenden Anmeldung werden Ergebnisse eines anderen, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitonin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procalcitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene entzündungs- und/- oder sepsisspezifische Biomarker von peptidischer bzw. proteinischer Natur zu ermitteln, deren Auftreten für bestimmte Formen von Entzündungen und/oder Sepsis charakteristisch ist und die daher eine spezifische, ggf. organspezifische Sepsis- bzw. Entzündungsdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/- oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol.18, 2000, 917-918. Angesichts der bisherigen eher enttäuschenden therapeutischen Ansätze besteht ein hohes Interesse daran, weitere möglichst entzündungs- bzw. sepsisspezifische endogene Biomoleküle zu identifizieren, die auch als therapeutische Targets neue Erfolgsaussichten für die Entzündungsbekämpfung eröffnen.

Die vorliegende Erfindung beruht darauf, dass sich in Primaten und Menschen bei Sepsis im cytoplasmatischen Gehirngewebe das stark überexprimierte Protein LASP-1, bzw. ein Protein, das mit diesem innerhalb der ersten 200 Aminosäuren identisch ist, in einer löslichen Form nachweisen läßt, und zwar im Unterschied zu Gesunden bzw. Unbehandelten, bei denen es nicht oder nur in Konzentrationen an der analytischen Nachweisgrenze gefunden wird, und auch im Unterschied zu anderen Körpergeweben septischer Primaten-Versuchstiere, was dieses Protein für die Entzündungsdiagnostik und Infektions- bzw. Sepsisdiagnostik mit Gehirnbeteiligung geeignet erscheinen läßt.

Die Erfindung beruht ferner darauf, dass festgestellt wurde, dass auch in der Zirkulation von menschlichen Sepsispatienten, jedoch auch von Alzheimer-Patienten, eine deutlich erhöhte LASP-1 Immunreaktivität messbar ist, was den Wert der auf der Basis von Gehirnextrakten von Primaten gewonnenen Erkenntnisse untermauert bzw. stark erhöht.

Die Verwendungen in der Diagnostik, die sich aufgrund des erstmals nachgewiesenen gehirnspezifischen Auftretens von LASP-1 bei der experimentellen Simulation von Sepsis, sowie aus dem Nachweis einer deutlich erhöhten LASP-1 Immunreaktivität in der Serum-oder Plasmaproben ergeben, werden nachfolgend noch näher beschrieben.

Die Ansprüche 1 bis 11 definieren die sich ergebenden neuen Verfahren gemäß der vorliegenden Erfindung, und bevorzugter Ausführungsformen davon, näher.

Wie nachfolgend im experimentellen Teil noch näher ausgeführt wird, war Ausgangspunkt der Erfindung die Feststellung, dass nach experimenteller Auslösung einer künstlichen Sepsis in Pavianen durch Endotoxinverabreichung (LPS aus Salmonella Typhimurium) und 2D-gelektrophoretischer Aufarbeitung von Gehirngewebe der behandelten Tiere als eines der nur bei den behandelten Tieren identifizierbaren Produkte ein Protein gefunden wurde, das weitgehend, und zwar wenigstens über den Abschnitt der Aminosäuren 1 bis 200, mit dem bekannten Protein LASP-1 identisch war, wobei das Protein außerdem nur im Gehirngewebe in erheblich erhöhter Konzentration beobachtet wurde. Zu seiner Identifizierung wurde ein neuer Proteinspot, der nur im Gehirngewebe der behandelten Tiere gefunden wurde und gemäß Gelektrophorese ein Molekulargewicht von etwa 36 ± 3 kDA und einen isoelektrischen Punkt von ca. 6,6-7,0 aufwies, aus dem Elektrophoresegel isoliert und durch Trypsinverdauung in Bruchstücke zerlegt, die auf an sich bekannte Weise massenspektrometrisch analysiert wurden. Durch Vergleich mit dem bekannten Massenspektrum des trypsinbehandelten Proteins LASP-1 wurde das Protein des isolierten Spots als eines identifiziert, das wenigstens über weite Abschnitte mit dem Protein LASP-1 identisch war.

Die Identifizierung des in dem Proteinspot gefundenen Produkts wird im experimentellen Teil näher beschrieben, wobei die beschriebene Identifizierung nach anerkannten Interpretationsgrundsätzen als sichere Identifizierung eines Produkts mit einer Aminosäuresequenz des Proteins LASP-1 anzusehen ist.

Das rechnerisch ermittelte Molekulargewicht der Peptidkette von LASP-1 (vgl. SEQ ID NO:1; 261 Aminosäuren) beträgt 29 717 Dalton. LASP-1 liegt jedoch physiologisch in posttranslational prozessierter Form (glykosyliert und phosphoryliert) vor. So wurde das später als LASP-1 bezeichnete Produkt ursprünglich als ein 40 kDA cAMP-abhängiges Phosphoprotein ("pp40") in Magenwandzellen identifiziert (vgl. C.S. Chew et al., Journal of Cell Science 113, 2035-2045 (2000); C.S. Chew et al., Am.J.Physiol. 275 (Cell Physiol. 44): C56-C67, (1998)).

Die Aminosäuresequenz des als LASP-1 bezeichneten Phosphoproteins wurde ursprünglich als die des putativen Expressionsprodukts der cDNA eines in humanen Brustkrebs-Zellinien überexprimierten Gens "MLN 50" aus dem Bereich q11-q21.3 des Chromosoms 17 ermittelt (C.Tomasetto at al., FEBS Letters 373 (1995): 245-249; C.Tomasetto et al., Genomics 28: 367-376 (1995); I.Bièche et al., Cancer Res. 56: 3886-3890 (1996)). Der Name LASP-1 geht darauf zurück, dass in diesem Protein erstmals eine sog. LIM-Domäne (eine aus verschiedenen Proteinen bekannte Zn-bindende Domäne; vgl. A.Hammarström et al., Biochemistry 1996, 35, 12723-12732) mit einer aus der rezeptorunabhängigen sog. src-Kinase, einer Tyrosin-Kinase, bekannten Domäne (SH3) kombiniert ist (LIM and SH3 Protein); vgl. auch Brian K. Kay et al., FASEB J. 14, 231-241 (2000).

LASP-1-mRNA ist ubiquitär in normalen Zellen nachweisbar (z.B. Prostata, Leber, Muskel, Gehirn, verschiedenen ZellTypen) und wird in einem gewissen prozentualen Anteil von Brustkrebsfällen überexprimiert. Es wurde festgestellt, dass LASP-1 ein Actin-bindendes Protein ist, für das angenommen wird, dass es zu den Cytoskelett-assoziierten Proteinen zu zählen ist und für die Zellform und -beweglichkeit sowie möglicherweise die Signalübertragung von Bedeutung ist. Es ist auch in Nervenenden nachweisbar. LASP-1 wird durch Detergentien nur schwer solubilisiert. In vitro hat sich LASP-1 als Substrat erwiesen, dass sich hervorragend mit der cAMP-abhängigen Serin/Threonin-Kinase PKA phosphorylieren läßt. LASP-1 weist in unterschiedlichen Epithel-Zelltypen unterschiedliche Expressionsmuster auf (vgl. C.S. Chew et al., Journal of Cell Science 113, 2035-2045 (2000); C.S. Chew et al., Am.J.Physiol. 275 (Cell Physiol. 44): C56-C67, (1998)).

Das Auftreten von LASP-1 bzw. eines weitgehend damit übereinstimmenden Proteins in überexprimierter Form im Gehirngewebe bei Sepsis ist von hohem wissenschaftlichen, diagnostischen und möglicherweise auch therapeutischen Interesse. Die Tatsache, dass LASP-1 ein Kinase-Substrat und Phosphoprotein ist, verleiht ihm eine gewisse Ähnlichkeit mit den sogenannen tau-Proteinen, die bei der Alzheimer Krankheit eine Rolle insbesondere für diagnostische Zwecke spielen (vgl. z.B. G.A.Jicha et al., Journal of Neurochemistry,69., 2087-2095 (1997) und die darin zitierte Literatur; sowie EP 673 418; EP 737 208; EP 772 634; EP 610 330; und EP 618 968).

Es ist nunmehr gut bekannt, dass die Alzheimer Krankheit als gehirnspezifischer Entzündungsprozess angesehen werden kann, ohne dass damit eine Aussage bezüglich Krankheitsursache oder Krankheitsfolge gemacht werden soll (Lih-Fen Lue et al., GLIA 35:72-79, 2001; Michael Hüll et al., DDT Vol.4, No.6: 275-282; (June 1999); F.Licastro et al., Journal of Neuroimmunologie 103 (2000), 97-102; Neuroinflammation Working Group: Haruhiko Akiyama et al., Neurobiology of Aging 21 (2000) 383-421; Michael Hüll et al., Exp.Opin.Invest.Drugs (2000) 9(4):671-683; M.Hüll et al., Eur Arch Psychiatry Clin Neurosci (1996) 246:124-128; J.Rhodin et al., Annals New York Academy of Sciences, 199x, 345-352). Die Tatsache, dass das Phosphoprotein LASP-1 bzw. ein damit innerhalb der ersten 200 Aminosäuren übereinstimmendes Protein unter experimentellen Bedingungen, die eine Sepsis bzw. ein sepsisähnliches systemisches Entzündungsgeschehen simulieren, gehirnspezifisch in überexprimierter Form nachweisbar ist, machte es sehr wahrscheinlich, dass auch bei der Alzheimer Krankheit eine Überexpression von Lasp-1 bzw. einer bestimmten Expressionsform von LASP-1 beobachtbar ist und die Bestimmung von LASP-1 daher auch für die Alzheimer-Diagnostik Bedeutung erlangen wird. Anschließend durchgeführte immundiagnostische Bestimmungen einer Immunreaktivität in Seren von Sepsis- und Alzheimer-Patienten, die der Sequenz der ersten 200 Aminosäuren von LASP-1 zugeordnet werden konnte, bestätigten diese Vermutung experimentell in überzeugender, eindrücklicher Weise.

Die internationale Patentanmeldung WO0185986 beschreibt ein Verfahren zur Identifizierung von Modulatoren der Bindung von LASP-1 an eine der Untereinheiten einer Isoform der Phosphatidylinositol-3-Kinase (PI3Kδ ), die hauptsächlich in Leukozyten exprimiert wird. Mit diesen Modulatoren soll eine Schädigung durch überschießende Entzündungsreaktionen (u.a. Sepsis) vermieden werden.

Es war aufgrund der bisher zugänglichen Befunde nicht zu erwarten, das die physiologischen Konzentrationen von LASP-1 in einer Serum- oder Plasmaprobe als Folge einer Sepsis und/oder gehirnspezifischen Entzündung wie Alzheimer signifikant und nachweisbar verändert sind und daher eine Bestimmung der LASP-1-Konzentrationen in solchen Proben im Rahmen eines Sepsisgeschehens oder einer entzündlichen Gehirnerkrankung diagnostisch von Interesse sein könnte.

Der erfindungsgemäße Nachweis von vergleichsweise hohen LASP-1-Konzentrationen im Gehirngewebe von Primaten, bei denen durch Toxinverabreichung eine künstliche Sepsis ausgelöst wurde, bei gleichzeitiger Unmöglichkeit, LASP-1 in ansonsten völlig gleich behandelten Proben von Kontrolltieren, oder in anderen Gewebeproben der septischen Tiere, zu erkennen, und der nachfolgende immundiagnostische Nachweise in Serum- oder Plasmaproben von menschlichen Patienten, ist hoch signifikant. Da das Auftreten nur bei den behandelten Tieren zu beobachten war, und zwar bereits relativ kurze Zeit nach der Sepsisauslösung durch Toxinverabreichung, ist es möglich, diese Tatsache zur Schaffung eines vielversprechenden diagnostischen Verfahrens zur Diagnose von Sepsis bzw. sepsisähnlichen, systemischen Infektionen durch Bestimmung von LASP 1-Immunreaktivität zu nutzen. Von besonderem Interesse ist außerdem die nachgewiesene Eignung von LASP-1 als diagnostischer Marker und Prognosemarker für die Alzheimer Krankheit.

Die Bestimmung von LASP-1 kann dabei nach irgendeinem beliebigen geeigneten Nachweisverfahren erfolgen, wobei jedoch die Bestimmung in einer Serum- oder Plasmaprobe , eines Patienten auf immundiagnostischem Wege (mittels eines Immunoassays) unter Verwendung geeigneter selektiver Antikörper unter praktischen Gesichtspunkten am vorteilhaftesten erscheint.

Aufgrund der Tatsache, dass bei einer experimentellen Sepsisauslösung im Gehirn von Primaten sowie anschließend auch in einer Serum- oder Plasmaprobe von menschlichen Patienten erstmals ein erhöhtes Auftreten von LASP-1 bzw. eines damit innerhalb der ersten 200 Aminosäuren identischen Proteins nachgewiesen werden konnte, wird somit die Möglichkeit geschaffen, die LASP-1 Immunreaktivität insbesondere für diagnostische Zwecke im Zusammenhang mit Sepsis und sepsisähnlichen systemischen Infektionen, sowie der Alzheimer Krankheit und Herzinfarkten, zu nutzen. Dafür können LASP-1 oder geeignete Teilpeptide davon oder Epitope oder Epitopkombinationen ggf. auch nach Verfahren, die inzwischen zum Stand der Technik gehören, synthetisch oder gentechnologisch gezielt hergestellt werden. LASP-1-Teilpeptide, ggf. in markierter Form, können auch als Kalibratoren, Tracer und Kompetitoren für bestimmte Assayformate für immundiagnostische Bestimmungen benötigt und dafür wie erläutert hergestellt werden. Bestimmte derzeit bevorzugte konkrete Ausführungsformen werden im nachfolgenden experimentellen Teil noch näher beschrieben.

Ferner können LASP-1-Fragmente bzw. geeignete Teilsequenzen davon nach bekannten Verfahren des modernen Standes der Technik auch zur Erzeugung spezifischer polyklonaler oder auch monoklonaler Antikörper verwendet werden, die als Hilfsmittel für die diagnostische Bestimmung von LASP-1 in Körperflüssigkeiten eines Patienten und/oder auch als potentielle therapeutische Mittel geeignet sind. Bestimmte derzeit bevorzugte konkrete polyklonale affinitätsgereinigte anti-LASP-1 Antikörper werden im nachfolgenden experimentellen Teil noch näher beschrieben. Die Erzeugung geeigneter monoklonaler Antikörper gegen bekannte Peptid-Teilsequenzen gehört heute zum allgemeinen Stand der Technik und muss nicht besonders beschrieben werden. Ferner ist auch ausdrücklich die Antikörpererzeugung unter Anwendung von Techniken der direkten genetischen Immunisierung mit einer entsprechenden DNA zu erwähnen. Es liegt somit im Rahmen der vorliegenden Erfindung, zur Immunisierung z.B. eine cDNA von LASP-1 oder LASP-1-Fragmenten zu verwenden, da es sich in der Vergangenheit gezeigt hat, dass bei der Anwendung derartiger Immunisierungstechniken das Spektrum der gewinnbaren Antikörper erweitert werden kann. Es können aber auch bereits bekannte Antikörper gegen LASP-1 verwendet werden (vgl. z.B. V.Schreiber et al., Molecular Medicine 4: 675-687, 1998; C.S. Chew et al., Am.J.Physiol. 275 (Cell Physiol. 44): C56-C67, (1998)).

Da im Rahmen der vorliegend beschriebenen Arbeiten im Gel des 2D-Elektrophorese ein LASP-1-Produkt mit einer molaren Massen von ca. 36 kDa gefunden wurde, das eine für die beschriebene Bestimmung ausreichende Löslichkeit aufweist, war nicht auszuschließen, dass das gefundene LASP-1-Produkt ein vom eigentlichen, in der Literatur beschriebenen LASP-1 abweichendes Expressionsprodukt, z.B. das Produkt eines alternativen Splicings, und/oder eine bestimmte Art der Glykosylierung und/oder Phosphorylierung aufweist, die ihm die beobachtete Löslichkeit verleiht und aufgrund derer es sich von dem aus der Magenwand isolierten 40 kDa-Produkt signifikant unterscheidet. Es wurde daher von Anfang der Untersuchungen an als im Rahmen der vorliegenden Erfindung liegend angesehen, die Bestimmung von LASP-1 mit Hilfe eines spezifischen Assays durchzuführen, bei dem spezifische Teilsequenzen und/oder ggf. auch Glykosylierungs- und/oder Phosphorylierungsmuster erkannt werden. Die Herstellung von Antikörpern, insbesondere monoklonalen Antikörpern, die spezifische Glykosylierungen und/oder Phosphorylierungen erkennen, ist grundsätzlich bekannt und z.B. im Zusammenhang mit der Bestimmung bestimmter Phosphorylierungsformen der sogenannten tau-Proteine im Rahmen der Alzheimer-Diagnostik beschrieben.

Bei der immunologischen Bestimmung von LASP-1 kann dabei grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851. Variationen der beschriebenen Techniken und/oder weitere Immunisierungstechniken kann der Fachmann einschlägigen Standardwerken und Veröffentlichungen entnehmen und sinngemäß anwenden.

LASP-1 oder ggf. LASP-1-Fragmente oder partiell sequenzidentische Produkte wie LAP-1 können aufgrund der vorliegenden Ergebnisse als Immunreaktivität bzw. spezifische Markerpeptide (Biomarker) zum diagnostischen Nachweis und zur Verlaufskontrolle von Sepsis, systemischen Infektionen vom Sepsistyp, Herzinfarkten und Entzündungen des Gehirns wie der Alzheimer-Krankheit dienen. Wie die Bestimmung von Procalcitonin kann dabei die Bestimmung von LASP-1 zur differentialdiagnostischen Früherkennung und zur Erkennung sowie für die Erstellung einer Verlaufsprognose, zur Beurteilung des Schweregrads und zur therapiebegleitenden Verlaufsbeurteilung von Entzündungen und Infektionen erfolgen, wobei man bei einem derartigen Verfahren in einer Serum- oder Plasma-Probe eines Patienten den Gehalt von LASP-1 bestimmt und aus der festgestellten Anwesenheit und/oder Menge von LASP-1 auf das Vorliegen einer Sepsis, Herzinfarktes oder der Alzheimer Krankheit schließt und das erhaltene Ergebnis mit dem Schweregrad der Sepsis, des Herzinfarktes und der Alzheimer Krankheit korreliert und ggf. die Behandlungsmöglichkeiten und/oder die Behandlungsaussichten abschätzt.

Besonders interessant erscheint eine Bestimmung von LASP-1 im Rahmen einer Multiparameter-Bestimmung, bei der gleichzeitig mindestens ein weiterer Entzündungs- oder Infektionsparameter bestimmt wird und bei der ein Messergebnis in Form eines Satzes von mindestens zwei Messgrößen gewonnen wird, der zur Feindiagnostik der Entzündung oder Infektion ausgewertet wird. Als derartige weitere Entzündungs- oder Infektionsparameter sind solche anzusehen, die aus der Gruppe der teilweise bekannten oder in den älteren bzw. parallelen Patentanmeldungen der Anmelderin offenbarten Parameter ausgewählt sind, die besteht aus Procalcitonin, CA 125, CA 19-9, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen, der Glycin-N-Acyltransferase (GNAT), der Carbamoylphosphat Synthetase 1 (CPS 1) und deren Fragmenten und dem C-reaktiven Protein (CRP) oder Fragmenten aller genannten Proteine. Es ist vorteilhaft, die Multiparameter-Bestimmung als Simultanbestimmung mittels einer Chiptechnologie-Messvorrichtung oder einer immunchromatographischen Messvorrichtung durchzufüren, bei der die Auswertung des mit der Messvorrichtung gewonnenen komplexen Messergebnisses mit Hilfe eines Computerprogramms erfolgt.

Bei einer bevorzugten Ausführungsform wird das Verfahren als heterogener Sandwich-Immunoassay durchgeführt, bei dem einer der Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während der andere Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Die beiden Antikörper können auch im Falle heterogener Sandwich-Immunoassays Teile eines Nachweissystems der nachfolgend im Zusammenhang mit homogenen Assays beschriebenen Art aufweisen.

Es liegt somit im Rahmen der vorliegenden Erfindung, das erfindungsgemäße Verfahren auch als Schnelltest auszugestalten.

Das erfindungsgemäße Verfahren kann ferner als homogenes Verfahren ausgestaltet werden, bei dem die aus den beiden Antikörpern und dem nachzuweisenden LASP-1 gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TRACE^{®} (Time Resolved Amplified Cryptate Emission) bzw. KRYPTOR^{®} angebotene Technologie zu verweisen, die die Lehren der o.g. Anmeldungen umsetzt.

Nachfolgend wird die Auffindung und Identifizierung von LASP-1 und dessen Bestimmung in biologischen Flüssigkeiten (Seren bzw. Plasmen) von Patienten mit verschiedenen Krankheiten in näheren Einzelheiten geschildert. Die Figuren zeigen:
- Fig. 1: Ansichten von 2D-Elektrophoresegelen, die einen Vergleich der Spotmuster der cytoplasmatischen Gehirnproteine eines gesunden Pavians (A) mit denen der Gehirnproteine eines Pavians 5h nach einer durch LPS-Verabreichung induzierten Sepsis (B) ermöglichen. Der Pfeil zeigt die Position des erfindungsgemäßen sepsisspezifischen Produkts (LASP-1) an, das in Darstellung (B) durch einen Kreis hervorgehoben ist;

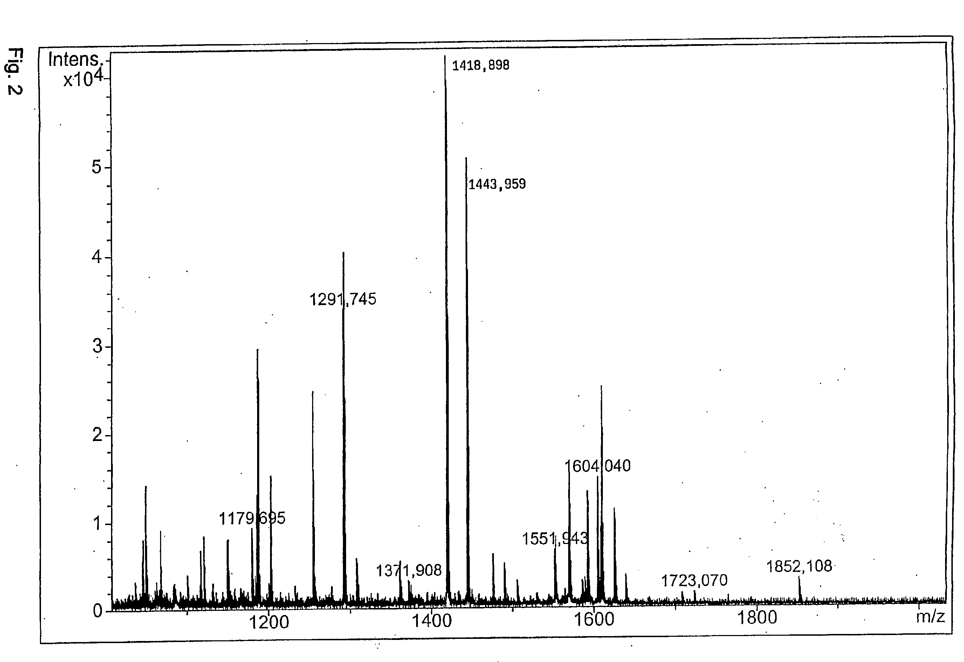

- Fig. 2: das Massenspektrum des aus dem Gel der 2D-Gelelek- trophorese isolierten, trypsinverdauten Produkts.
- Fig. 3: eine vergleichende Darstellung der Ergebnisse der Messung der LASP-1 (1-200) Immunreaktivität unter Verwendung von Immunoassays mit verschiedenen Paaren von drei Antikörpern, die in festphasenge- bundener bzw. markierter Form eingesetzt wurden und die Aminosäuresequenzen 121-137, 147-159 bzw. 170-187 von LASP-1 erkannten. Es ist zu erkennen, dass mit allen Paaren weitgehend identische Ergeb- nisse erhalten wurden.
- Fig. 4: die Ergebnisse einer immundiagnostischen Bestim- mung einer LASP-1 (1-200) Immunreaktivität in Seren von 294 gesunden Kontrollpersonen und von 96 Sepsispatienten.
- Fig. 5: die Ergebnisse einer immundiagnostischen Bestim- mung einer LASP-1 (1-200) Immunreaktivität in Seren von 294 gesunden Kontrollpersonen und von 80 Alzheimer-Patienten.
- Fig. 6: die Ergebnisse einer immundiagnostischen Bestim- mung einer LASP-1 (1-200) Immunreaktivität in Seren von 294 gesunden Kontrollpersonen und von 20 Herzinfarkt-Patienten.

### EXPERIMENTELLER TEIL

### 1. Infektionssimmulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 µg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Einfrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Nach einer anschließenden 1-stündigen Zentrifugation bei 100.000 g und +4°C wurde der erhaltene Überstand gewonnen und bis zur weiteren Verarbeitung bei -80°C gelagert.

### 2. Proteomanalyse unter Verwendung cytoplasmatischer Gehirnproteine von Pavianen.

Cytoplasmatische Gehrinzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Gehirnextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

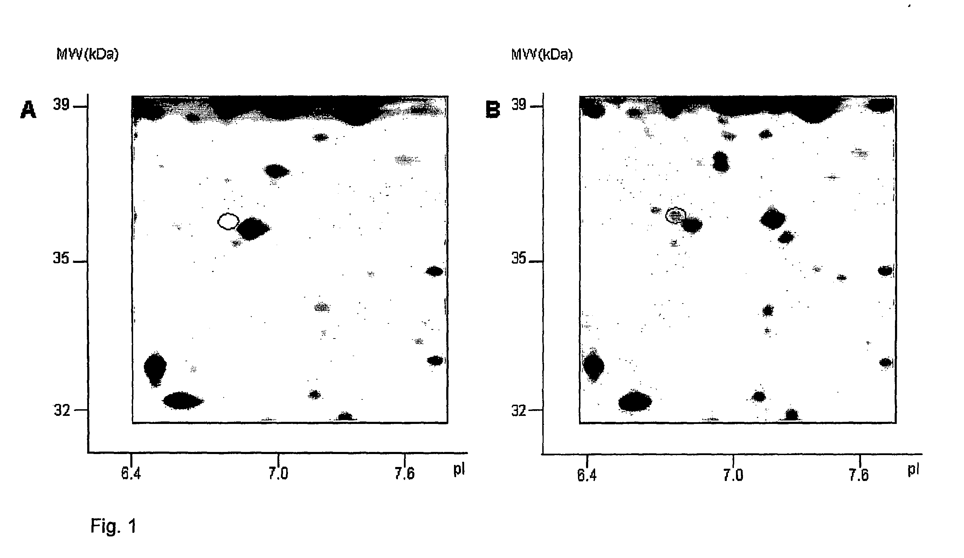

Zur Auswertung wurden die Proteinspotmuster der Proben behandelter Tieren mit den Proteinspotmustern verglichen, die aus Gehirngewebeproben unbehandelter Tiere resultierten. Ferner wurden die Proteinspotmuster aus dem Gehirngewebe behandelter Tiere auch mit denen anderer Gewebe derselben behandelten Tiere verglichen (die Ergebnisse sind nicht im Einzelnen gezeigt). Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei der zusätzliche Proteinspot in (B) einem neuen Protein entspricht, dessen Position durch einen Pfeil und einen Kreis hervorgehoben ist.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262). Die für die weitere Analyse vorselektierten Proteinspots wurden aus dem Gel ausgeschnitten, unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol.26, 1, 2001, 54-61; beschrieben und diskutiert werden. Die trypsinverdauten Proben wurden einer MALDI-TOF Massenspektrometrie unterzogen.

### 3. Identifizierung von LASP-1

Wie in den Figuren 1(A) und 1(B) gezeigt ist, findet sich in Gehirngewebeextrakten von Pavianen, denen eine LPS-Injektion verabreicht worden war, u.a. ein Spot eines Proteins, für das aufgrund der Gelektrophoresedaten im Vergleich mit Markersubstanzen mit bekanntem Molekulargewicht ein Molekulargewicht von ca. 36 ± 3 kDa abgeschätzt wurde, während aus der relativen Position des Proteins aus der ersten Dimension ein isoelektrischer Punkt von ca. 6,6-7,0 ermittelt wurde.

Bei der Massenanalyse des tryptischen Verdaus des neuen Proteinspots wurde für 21 Bruchstücke unter Rückgriff auf Datenbankinformationen der Versuch einer Zuordnung zu Peptiden vorgenommen. Im Ergebnis entsprachen zwölf der erhaltenen Zuordnungen Bruchstücken der Proteinkette des Phosphoproteins LASP-1 (SEQ ID NO:1). Die sog. "Sequence Coverage" betrug 38%, was nach den anerkannten fachüblichen Kriterien als eindeutige Identifizierung des in dem Spot enthaltenen Proteins als Proteinkette von LASP-1 anzusehen ist.

So konnten z.B. die folgenden Massen Bruchstücken der LASP-1-Proteinkette (SEQ ID NO.1) zugeordnet werden: 1202,714; 1254,615; 1291,745; 1360,813; 1418,898; 1443,959; 1489,826; 1551,943; 1604,040; 1608,960; und zwar war die Zuordnung gemäß MS-Fit 3.3.1. Protein Prospector 3.4.1., Database NCBInr.7.25.2002 wie folgt:

Die Masse 1202,714 (MH⁺) entspricht der Sequenz QQSELQSQVR (SEQ ID NO:2). Das theoretische Molekulargewicht dieses Fragments beträgt 1201,604. In der Aminosäuresequenz von LASP-1 findet sich diese Sequenz an den Positionen 76-95 wieder.

Die Masse 1254,615 entspricht der Sequenz ACFHCETCK (SEQ ID NO:3). Das theoretische Molekulargewicht dieses Fragments beträgt 1253,498. In der Aminosäuresequenz von LASP-1 findet sich diese Sequenz an den Positionen 28-36 wieder.

Die Masse 1291,745 entspricht der Sequenz KPYCNAHYPK (SEQ ID NO:4). Das theoretische Molekulargewicht dieses Fragments beträgt 1290.617. In der Aminosäuresequenz von LASP-1 findet sich diese Sequenz an den Positionen 50-59 wieder.

Die Masse 1360,813 entspricht der Sequenz VNCLDKFWHK (SEQ ID NO:5). Das theoretische Molekulargewicht dieses Fragments beträgt 1359,675. In der Aminosäuresequenz von LASP-1 findet sich diese Sequenz an den Positionen 18-27 wieder.

Die Masse 1418,898 entspricht der Sequenz GFSVVADTPELQR (SEQ ID NO:6). Das theoretische Molekulargewicht dieses Fragments beträgt 1417,719. In der Aminosäuresequenz von LASP-1 findet sich diese Sequenz an den Positionen 97-109 wieder.

Die Masse 1443,959 entspricht der Sequenz LKQQSELQSQVR (SEQ ID NO:7). Das theoretische Molekulargewicht dieses Fragments beträgt 1442,783. In der Aminosäuresequenz von LASP-1 findet sich diese Sequenz an den Positionen 74-85 wieder.

Die Masse 1489,826 entspricht der Sequenz MGPSGGEGMEPERR (SEQ ID NO:8). Das theoretische Molekulargewicht dieses Fragments beträgt 1488,644. In der Aminosäuresequenz von LASP-1 findet sich diese Sequenz an den Positionen 131-144 wieder.

Die Masse 1551,943 wurde der Sequenz TGDTGMLPANYVEAI (SEQ ID NO:9) zugeordnet. Das theoretische Molekulargewicht dieses Fragments beträgt 1550,728. In der Aminosäuresequenz von LASP-1 findet sich eine solche Sequenz an den Positionen 247-261 wieder.

Die Masse 1604,040 entspricht der Sequenz GKGFSVVADTPELQR (SEQ ID NO:10). Das theoretische Molekulargewicht dieses Fragments beträgt 1602,836. In der Aminosäuresequenz von LASP-1 findet sich diese Sequenz an den Positionen 95-109 wieder.

Die Masse 1608,960 entspricht der Sequenz QSFTMVADTPENLR (SEQ ID NO:11). Das theoretische Molekulargewicht dieses Fragments beträgt 1607,761. In der Aminosäuresequenz von LASP-1 findet sich diese Sequenz an den Positionen 60-73 wieder.

Neun der zehn beschriebenen identifizierten Fragmente finden sich im Abschnitt der Aminosäuren 1-200 von LASP-1 wieder, d.h. demjenigen Protein-Abschnitt, der vor der sog. SH3-Domäne liegt, die bei Aminosäure 202 beginnt. Eine solche Übereinstimmung gilt nach anerkannten Interpretationsgrundsätzen als sichere Identifizierung des Produkts aus dem Proteinspot als ein wenigstens im Bereich der Aminosäuren 1-200 mit LASP-1 übereinstimmenden Protein. Das Molekularge-wicht von unphosphoryliertem LASP-1 (261 Aminosäuren; vgl. SEQ ID NO:1) beträgt jedoch nur 29 717 Dalton. Aus der Literatur ist es jedoch bekannt, dass LASP-1 bei gelektrophoretischen Molmasse-Bestimmungen ein irreguläres Verhalten zu zeigen scheint, so dass die gefundenen Abweichungen der Molmassen nicht unbedingt im Widerspruch dazu stehen, die gefundene Substanz als LASP-1 mit der Aminosäuresequenz gemäß SEQ ID NO:1 zu identifizieren.

Es ist jedoch ebensowenig völlig auszuschließen, dass sich das nachgewiesene Produkt von dem genannten LASP-1 unterscheidet, z.B. das Produkt eines alternativen Splicings ist. Es wurden daher zur Auslotung der Möglichkeit, dass es sich um ein anderes verwandtes Expressionsprodukt gehandelt haben könnte, eine Folgeuntersuchungen vorgenommen, die die Möglichkeit einer Zuordnung des gefundenen Proteinspots zu einem nahe verwandten längeren Protein ausloten sollte. Diese unter Heranziehung von in cDNA-Datenbanken implizit vorhandenen Informationen durchgeführten Anschlußuntersuchungen führten zur Auffindung eines mit LASP-1 verwandten längeren Proteins, das das Produkt eines alternativen Splicings sein dürfte und anstelle der SH3-Domäne einen alternativen C-terminalen Aminosäurerest von 123 Aminosäuren aufweist. Das Protein weist die gleichen Aminosäuren 1-200 wie LASP-1 auf, so dass eine mit LASP-1 vergleichbare Immunreaktivität erwartet werden könnte. Dem genannten verwandten Protein wurde die Bezeichnung LAP-1 gegeben. Zu seiner Identifizierung finden sich nähere Angaben im nachfolgenden Abschnitt 5.

### 4. Immundiagnostische Bestimmung von LASP-1 Immunreaktivitäten in Seren von menschlichen Patienten

### 4.1. Immunoassays - Material und Methoden

### 4.1.1. Peptidsynthesen

Abgeleitet von der bekannten Aminosäuresequenz des humanen LASP-1 wurden drei Bereiche ausgewählt (Pos. 121-137: Peptidbereich 1; Pos. 147-159: Peptidbereich 2; Pos. 170-187: Peptidbereich 3;). Jeweils ergänzt um einen N-terminalen Cystein-Rest wurden die Bereiche nach Standardverfahren als lösliche Peptide chemisch synthetisiert, gereinigt, mittels Massenspektrometrie und Reversed Phase HPLC qualitätskontrolliert und in Aliquots lyophilisiert (Firma JERINI AG, Berlin, Deutschland). Die Aminosäuresequenzen der synthetischen Peptide lauten.

| | |
|---|---|
| Peptid PKE18 | CKYHEEFEKSRMGPSGGE (SEQ ID NO:13) |
| Peptid PQQ14 | CQDGSSYRRPLEQQ (SEQ ID NO:14) |
| Peptid PVK19 | CVYQQPQQQPVAQSYGGYK (SEQ ID NO:17) |

Auerdem wurde als Standardpeptide folgendes Peptid synthetisiert, das sich aus den Positionen 121-137, 147-159, 170-187 des LASP-1 gemäß SEQ ID NO:1 zusammensetzte:

| | |
|---|---|
| Peptid PKK54 | |

Für die Peptidauswahl waren folgende Überlegungen ausschlaggebend:

Das 261 Aminosäuren umfassende Protein LASP-1 enthält zwei potentielle Protein-Bindungsdomänen, die LIM-Domäne am N-Terminus (Pos. 5-56) , und die SH3-Domäne am C-Terminus (Pos. 202-261) (vgl. Tomasetto et al., a.a.O. 1995). Ob und welche Proteinbindungen LASP-1 über diese Domänen eingeht, ist bis heute unbekannt. Als weitere strukturelle Merkmale von LASP-1 sind zwei mögliche Actin-Bindungsdomänen beschrieben: Diese liegen direkt aufeinander folgend im Bereich der Pos. 61-133 (Schreiber et al., a.a.O. 1998), und es ist gezeigt worden, daß LASP-1 tatsächlich Actin binden kann (Schreiber et al., a.a.O., 1998). Die Bindung an Actin wird durch Phosphorylierung an den Positionen 99 und 146 beeinflußt (Chew et al, a.a.O., 2002).

Zur Entwicklung von Immunoassays für LASP-1 und zur Prüfung der Frage, ob LASP-1 auch außerzellulär, d.h. als Immunreaktivität in normalen und pathologischen humanen Serum-/Plasmaproben nachzuweisen ist, orientierten sich die Erfinder an den oben beschriebenen bekannten Informationen zur Struktur von LASP-1: Es wurden drei Peptide zur Immunisierung ausgewählt, die nicht im Bereich der LIM- und SH3-Domänen liegen, da diese Domänen in LASP-1 möglicherweise durch gebundenes Protein für Antikörper nicht zugänglich sein könnten. Desweiteren enthalten die drei ausgewählten Peptide nicht die beschriebenen Phosphorylierungsstellen. Für eines der Peptide wurde in Kauf genommen, daß es teilweise in einen möglichen Actin-Bindungsbereich fällt.

### 4.1.2. Konjugation und Immunisierung

Mittels MBS (m-Maleimidobenzoyl-N-hydroxysuccinimid Ester) wurden die Peptide PKE18, PQQ14 und PVK19 jeweils an das Trägerprotein KLH (Keyhole limpet hemocyanin) konjugiert (s. Arbeitsanleitung "NHS-Esters-Maleimide Crosslinkers" Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### 4.1.3. Reinigung der Antikörper

In einem 1-Schritt Verfahren wurden aus den Antiseren, die beginnend mit dem vierten Monat nach der Immuniserung gewonnen worden waren, die Peptid-spezifischen Antikörper präpariert.

Dazu wurden zunächst die Peptide Peptide PKE18, PQQ14 und PVK19 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf Antiseren gegen beide Peptide wurde wie folgt durchgeführt:

Die Peptid-Säulen wurden zunächst drei mal im Wechsel mit je 10 ml Elutionspuffer (50 mM Citronensäure, pH 2.2) und Bindungspuffer (100 mM Natriumphosphat, 0.1% Tween, pH 6.8) gewaschen. 50 ml der Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt . Die Durchläufe wurden verworfen. Anschließend wurde mit 250 ml Bindungspuffer proteinfrei (Proteingehalt des Wascheluats < 0,02 A280 nm) gewaschen. Auf die gewaschene Säulen wurde Elutionspuffer gegeben, und es wurden Fraktionen ä 1 ml gesammelt. Von jeder Fraktion wurde der Proteingehalt mittels der BCA-Methode (s. Arbeitsanleitung Firma PIERCE, Rockford, IL, USA) bestimmt. Fraktionen mit Proteinkonzentrationen > 0.8 mg/ml wurden gepoolt, mit PBS Puffer neutralisiert und damit auf Proteinkonzentrationen von etwa 1 mg/ml verdünnt. Die Ausbeuten betrugen: 45 mg anti-PKE18 Antikörper, 59 mg anti-PQQ14 Antikörper und 14 mg anti-PVK19 Antikörper.

### 4.1.4. Markierung

Zur Chemilumineszenzmarkierung der Antikörper wurden 100 µl des anti-PVK19 Antikörpers (1 mg/ml) bzw. 125 µl des anti-PQQ14 Antikörpers (0.8 mg/ml) mit je 10 µl MA70-Akridinium-NHS-Ester (1 mg/ml; Firma HOECHST Behring) versetzt und 15 Minuten bei Raumtemperatur inkubiert. Dann wurden je 400 µl 1 M Glycin zugesetzt und weitere 10 Minuten inkubiert. Anschließend wurden die Markierungsansätze über NAP-5 Gelfiltrationssäulen (Pharmacia) in je 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurden Gelfiltrations-HPLCs durchgeführt (Säule: Waters Protein Pak SW300). Die Proben wurden aufgetragen und bei einer Flußrate von 1 ml/min mit Laufmittel A chromatographiert. Mit einem Duchflußphotometer wurden die Wellenlängen 280 nm und 368 nm gemessen. Das Absorptionsverhältnis 368 nm/280 nm als Maß für den Markierungsgrad der Antikörper betrug für beide Antikörper am Peak 0.10. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, gesammelt.

### 4.1.5. Kopplung

Bestrahlte 5 ml Polystyrolröhrchen (Firma Greiner) wurden mit anti-PQQ14 Antikörper bzw. anti-PKE18 Antikörper wie folgt beschichtet: Die Antikörper wurden in 50 mM Tris, 100 mM NaCl, pH 7,8 zu einer Konzentration von 6.6 µg/ml verdünnt. In jedes Röhrchen wurden 300 µl dieser Lösung pipettiert. Die Röhrchen würden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

### 4.2. Durchführung und Auswertung der Immunoassays

### 4.2.1. Assayaufbau und Durchführung

Es wurde ein Assaypuffer folgender Zusammensetzung hergestellt:
100 mM Natriumphosphat, 150 mM NaCl, 5% Bovines Serum Albumin, 0.1% unspez. Schaf IgG, 0.1% Natrium Azid, pH 7.4

Als Standardmaterial diente ein chemisch synthetisiertes künstliches Peptid PKK54, das die Pos. 121-137, 147-159, 170-187 von LASP-1 (SEQ ID NO:15) enthält. Dieses Peptid wurde seriell in Pferdenormalserum (Firma SIGMA) verdünnt. Den so hergestellten Standards wurden Konzentrationen gemäß der Einwaage an Peptid zugeschrieben.

Meßproben waren EDTA-Plasmen oder Seren von augenscheinlich Gesunden, von Patienten mit Sepsis, von Patienten mit der Alzheimer'schen Krankheit und Patienten mit Herzinfarkt.

Es wurden drei Typen von Sandwichassays durchgeführt. Folgende Antikörperkombinationen kamen dabei zum Einsatz:
Röhrchen: anti-PKE18 / Tracer: anti-PVK19
Röhrchen: anti-PQQ14 / Tracer: anti-PKE18
Röhrchen: anti-PQQ14 / Tracer: anti-PVK19

Alle Assays wurden wie folgt durchgeführt:

In die Teströhrchen wurden je 50 µl Standard bzw. Probe sowie 150 µl Assaypuffer pipettiert. Es wurde zwei Stunden bei 22°C unter Schütteln inkubiert. Dann wurde 4 x mit je 1 ml Waschlösung (0.1% Tween 20) pro Röhrchen gewaschen. Dann wurde in jedes Röhrchen 200 µl Assaypuffer, enthaltend 0.5 Millionen RLU des MA70-markierten Tracer-Antikörpers, pipettiert. Es wurde zwei Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen.

Unter Verwendung der Software MultiCalc (Spline Fit) wurde die Konzentration an LASP-1 Immunreaktivität abgelesen.

### 4.2.2. Ergebnisse

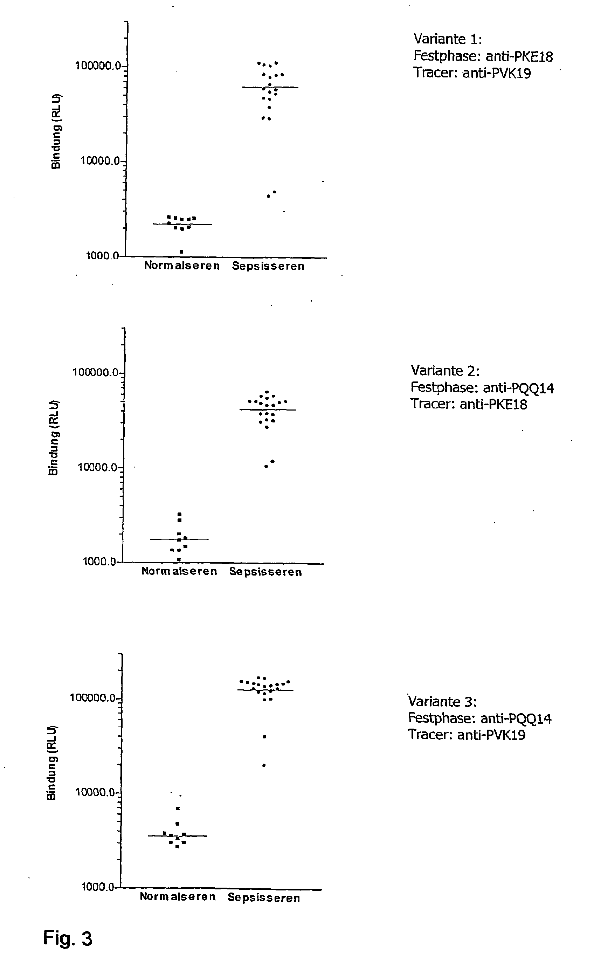

Überraschenderweise zeigte sich, daß mit allen drei oben beschriebenen Sandwich-Assays in Seren von Sepsispatienten eine deutliche und vergleichbare LASP-1 Immunreaktivität nachweisbar war (vgl. Figur 3). Diese Immunreaktivität lag deutlich höher als bei Kontrollproben von Gesunden. Das nachgewiesene Vorkommen von LASP-1 Immunreaktivität in der Blutzirkulation war vor dem Hintergrund des bekannten Wissens zu LASP-1 überraschend, da LASP-1 keine strukturellen Merkmale (wie eine Signalsequenz) enthält, die eine extrazelluläre Lokalisation des Moleküls vorhersagbar machen würden. Auch der Stand der Technik gibt keinerlei Hinweis auf eine solche Lokalisationsmöglichkeit. Vielmehr ist eine intrazelluläre Lokalisation in Zellmembran-Extensionen beschrieben (Schreiber et al., a.a.O., 1998).

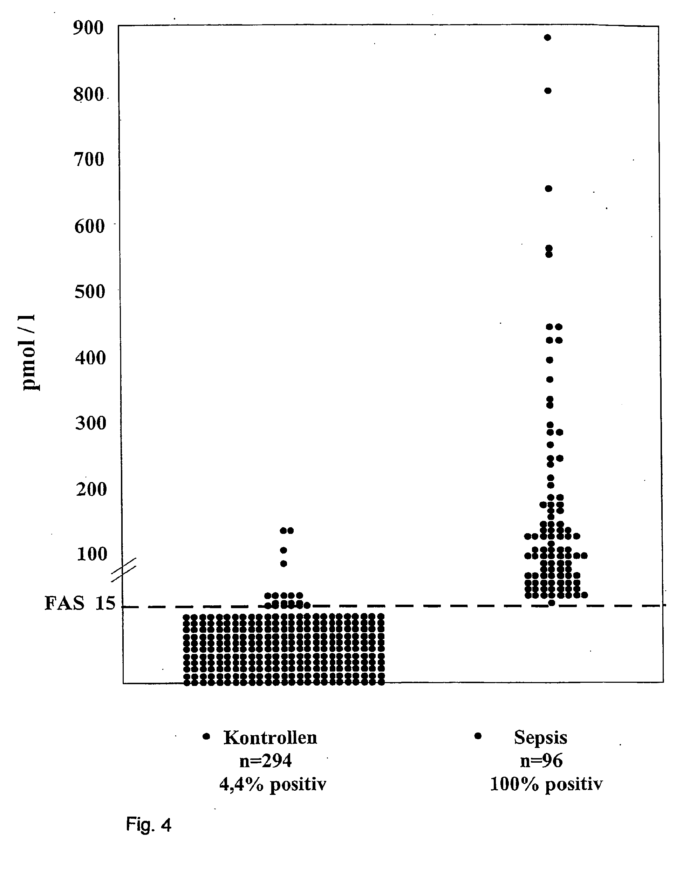

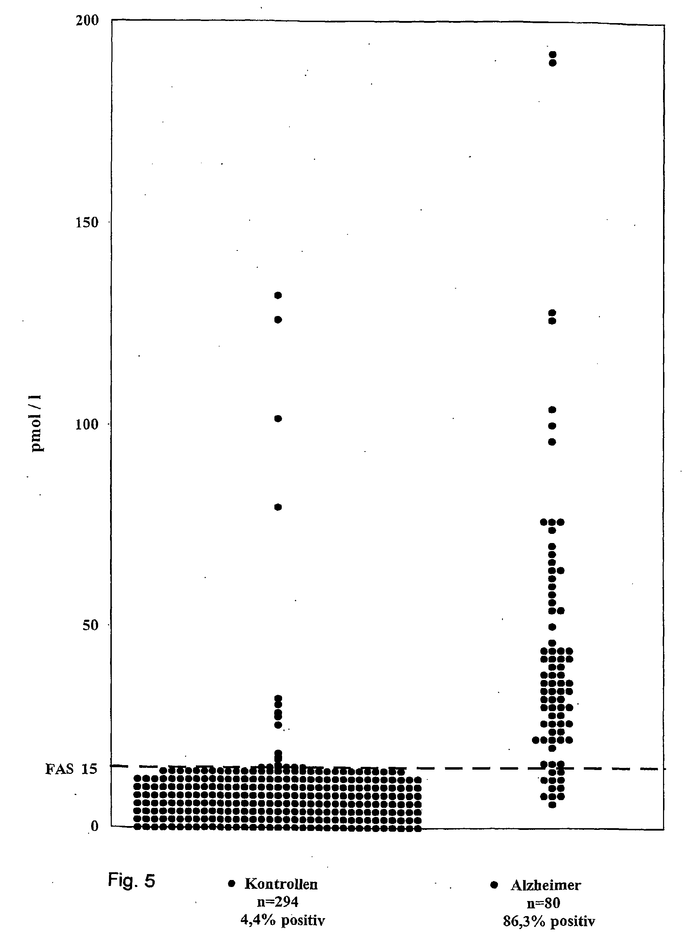

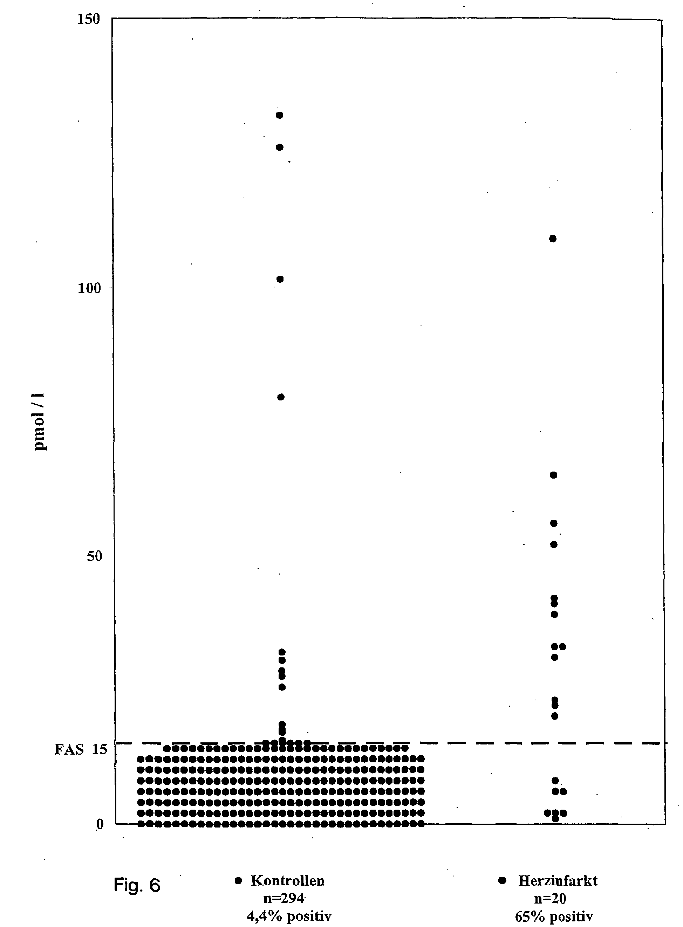

Mit einem der Assays (Röhrchen: anti-PQQ14 / Tracer: anti-PVK19) wurden umfangreichere Vermessungen durchgeführt. Zur Standardisierung dienten dabei Verdünnungen von Standardpeptid PKK54 in Pferdeserum. Dabei bestätigte sich die deutliche Erhöhung von LASP-1 Immunreaktivität bei einer größeren Fallzahl von Seren von Patienten mit Sepsis (Figur 4). Eine gegenüber gesunden Kontrollen ebenfalls erhöhte Immunreaktivität wurde auch für Patienten mit der Alzheimer'schen Krankheit (Figur 5) und, etwas weniger ausgeprägt, Patienten mit Herzinfarkt (Figur 6) nachgewiesen.

### 4.2.3. Hinweise zu Proteinbindungen von LASP-1 in Serum

Die Signalintensitäten für die einzelnen Serumproben - im Sandwichimmunoassay etwa proportional zur Analytkonzentration - korrelierten zwischen drei verschiedenen Assays (Figur 3). Diese Korrelation zeigt an, daß alle drei Epitope für die Bindung der verwendeten Antikörper bei allen vermessen Proben in ähnlicher Weise zugänglich sind. Für einen Analyten, der nicht an andere Proteine gebunden ist, wäre dies zu erwarten und nicht überraschend. Für LASP-1 allerdings sind mehrere Möglichkeiten beschrieben, Bindungen mit Proteinen einzugehen (a.a.O.). Eines der Epitope (Pos. 121-137) überlappt mit einer Actin-Bindungsstelle. Offenbar ist also diese Bindungsstelle in dem im Serum meßbaren Analyten mit LASP-1 Immunreaktivität nicht besetzt. Die beiden anderen Epitope (Pos. 147-159 und Pos. 170-187) liegen außerhalb von Proteinbindungsdomänen (Actin-Bindungsstellen, LIM, SH3). Ein direkter Einfluß auf Antikörperbindung an diese Epitope durch etwaig gebundene Proteine ist daher nicht zu erwarten. Jedoch ist nicht auszuschließen, daß es im Fall einer Proteinbindung zu einer indirekten sterischen Behinderung der Antikörperbindung kommen könnte. Offenbar ist dies aber nicht der Fall. Ob die LASP-1 Immunreaktivität im Serum tatsächlich frei vorliegt, ist durch die vorliegenden Analysen noch nicht bewiesen und ist ggf. durch weitere Untersuchungen festzustellen.

### 5. Identität der LASP-1 Immunreaktivität in Serum

Ein Vergleich der Aminosäuresequenzen der drei zur Erzeugung der anti-LASP-1 Antikörper verwendeten Peptide PKE18 (Pos. 121-137 in LASP-1; SEQ ID NO:13), PQQ14 (Pos. 147-159 in LASP-1; SEQ ID NO:14) und PVK19 (Pos. 170-187 in LASP-1; SEQ ID NO:17) mit den in der Datenbank GenBank eingetragenen Sequenzen aller bekannter humaner Proteine bzw. aus cDNAs abgeleiteten Proteine zeigte, daß neben LASP-1 (z.B. Accession Nr. BC012460) ein zweites Protein existiert, das diese Sequenzen enthält (Accession Nr. BC007560). Dieses wird im folgenden als LAP-1 bezeichnet. Ihm kann die Sequenz gemäß SEQ ID NO:16 zugeordnet werden. Die mit den oben beschriebenen Immunoassays gemessene Immunreaktivität könnte also sowohl von LASP-1 (SEQ ID NO:1) selbst als auch von LAP-1 (SEQ ID NO:16) herrühren.

Der Anmelderin ist kein Stand der Technik zu LAP-1 bekannt geworden, der über Einträge zur Primärstruktur in der Datenbank hinausgeht. Um nähere Information zur Struktur und Genese von LASP-1 im Vergleich zu LAP-1 zu erhalten, wurden daher von den Erfindern selbst weitergehende Analysen durchgeführt, deren Ergebnisse im folgenden zusammen gefaßt werden:

LASP-1 und LAP-1 resultieren offenbar aus einem unterschiedlichen Splicing des Primärtranskripts ein und desselben Gens: Die Sequenzen der cDNAs von LASP-1 und LAP-1 (Accession Nr. BC007560 und BC012460) wurden mit der Sequenz des Humangenoms verglichen. Dabei wurde ein zugehöriges Gen auf Chromosom 17, nahe 17q21, Region 39,023K-39,076K lokalisiert. Durch den Sequenzvergleich ließ sich eine Exon-Intron-Struktur des Gens ableiten. Die cDNAs für LASP-1 und LAP-1 resultieren aus dem Splicing von acht.Exons. Die Exons 2-5 unterscheiden sich für beide cDNAs nicht. Die Exons 6, 7 und 8 sind jedoch bei beiden cDNAs nicht identisch, überlappen sich aber. Exon 1 unterscheidet sich bei beiden cDNAs bezüglich der Länge am 5'-Terminus. Unklar bleibt, ob die unterschiedlichen Längen von Exon 1 beider cDNAs aus unterschiedlichen Transkriptionsinitiationen, oder aber aus unterschiedlichen Abbrüchen bei der reversen Transkription der mRNAs resultieren. Der translatierte Bereich reicht für beide cDNAs vom 3'-Bereich bis in den 5'-Bereich des Exons 7: Die Aminosäuresequenzen beider Translationsprodukte sind bis zur Aminosäure Prolin (Pos. 200) identisch, danach aber völlig unterschiedlich (vgl. SEQ ID NO:1 im Vergleich mit SEQ ID NO:16). LASP-1 umfaßt insgesamt 261 Aminosäuren, LAP-1 umfaßt 323 Aminosäuren. In LASP-1 beginnt die SH3 Domäne bei Position 202. Genau dieser SH3-Bereich ist in LAP-1 nicht vorhanden, sondern durch einen anderen Bereich unbekannter Funktion ersetzt. Das Fehlen der SH3 Domäne in LAP-1 führte zur Namensgebung (in "LASP-1" steht S für SH3 Domäne).

Sowohl zu LASP-1 als auch zu LAP-1 sind ESTs (expressed sequence tags) in der einschlägigen Datenbank GenBank "Human EST entries" vorhanden (z.B. BU553748 für LASP-1 und BG281978 für LAP-1). Das zeigt eindeutig, daß die oben beschriebenen Einträge BC007560 und BC012460 keine singulären Beobachtungen oder gar Artefakte sind, sondern weist vielmehr darauf hin, daß tatsächlich beide Splicing-Varianten exprimiert werden. Unter welchen Bedingungen das Gen jedoch in LASP-1 oder LAP-1 exprimiert wird, ist unbekannt.

Aufgrund der weitgehenden Sequenzidentität mit LASP-1 soll auch eine Bestimmung und/oder Mitbestimmung von LAP-1 als Bestimmung nach der vorliegenden Erfindung angesehen werden.

Wählt man für ein Bestimmungsverfahren die Reagenzien so aus, dass die Bestimmung zwischen LASP-1 und LAP-1 unterscheiden kann, z.B. indem man in einem Sandwichassay die Bindungspartner (festphasengebunden oder markiert) so wählt, dass wenigstens einer davon spezifisch ein Epitop im C-terminalen Teil oberhalb der Position 200 von LAP-1 oder LASP-1 erkennt, insbesondere oberhalb der Position 200 von LAP-1, kann man prüfen, ob die in einer biologischen Flüssigkeit nachgewiesene LASP-1 Immunreaktivität ganz oder teilweise auf LAP-1 zurück zu führen ist, und man kann LAP-1 auch allein neben LASP-1, oder neben der Gesamtmenge von LASP-1 und LAP-1, bestimmen und dadurch die klinische Relevanz einer Anwesenheit von LAP-1 für Sepsis und/oder die Alzheimer Krankheit genauer ausloten.

### Therapeutisches Potential der neuen Erkenntnisse

Welche Funktion die in pathologischen Bedingungen beobachtete stark erhöhte LASP-1 Immunreaktivität in der Blutzirkulation hat, ist offen. Schon der Umstand aber, daß eine Erhöhung mit einem pathologischen Zustand assoziiert ist, legt die Annahme nahe, daß die LASP-1 Immunreaktivität am molekularen Mechanismus der Krankheit oder aber dem körpereigenen Versuch der Bekämpfung der Krankheit beteiligt ist, möglicherweise dabei sogar eine Schlüsselrolle spielt. Es ist daher vielversprechend, weitere, auf den Erkenntnissen in der vorliegenden Anmeldung aufbauende Untersuchungen mit therapeutischer Zielrichtung durchzuführen. Je nachdem ob die LASP-1 Immunreaktivität eher schädlich oder nützlich ist, ergibt sich ein therapeutischer Nutzen entweder durch eine exogen herbeigeführte Minderung oder aber Erhöhung der LASP-1 Immunreaktivität.

Die erstmals festgestellten erhöhten LASP-1- bzw. ggf. LAP-1-Konzentrationen erlauben deren Verwendung als neue Biomarker für Sepsis, Alzheimer und auch im Zusammenhang mit Herzinfarkt. Das erstmal bewiesene krankheitsbegleitende Auftreten von LASP-1 in der Zirkulation von Sepsis-, Alzheimer- und Herzinfarkt-Patienten eröffnet ferner neue interessante Forschungsansätze zur Therapie der einschlägigen Erkrankungen.

## Patentansprüche

1. Diagnostisches in vitro Verfahren zur Früherkennung und Erkennung, für die Verlaufsprognose und die Beurteilung des Schweregrads und zur therapiebegleitenden Verlaufsbeurteilung von sepsisähnlichen systemischen Infektionen und Sepsis, Herzinfarkt und der Alzheimer Krankheit, bei dem man in einer Serum- oder Plasmaprobe eines Patienten die Anwesenheit und/oder Menge des Proteins LASP-1 (SEQ ID NO:1) oder eines Proteins, das mit diesem wenigstens im Bereich der ersten 200 Aminosäuren sequenzidentisch ist und eine dem genannten Bereich entsprechende Immunreaktivität aufweist, in freier und/oder proteingebundener Form oder posttranslational modifizierter Form bestimmt und aus der Anwesenheit und/oder Menge der bestimmten LASP-1 Immunreaktivität Schlüsse hinsichtlich des Vorliegens, des zu erwartenden Verlaufs, des Schweregrads oder des Erfolgs einer Therapie der sepsisähnlichen systemischen Infektion oder Sepsis, des Herzinfarkts oder der Alzheimer Krankheit zieht.

2. Verfahren nach Anspruch 1, **dadurch gekennzeichnet, dass** man bei der Bestimmung die Immunreaktivität eines Proteins, oder eines Fragments davon, bestimmt oder mitbestimmt, das als LAP-1 bezeichnet wird und die Aminosäuresequenz gemäß SEQ ID NO:16 aufweist.

3. Verfahren nach einem der Ansprüche 1 oder 2, **dadurch gekennzeichnet, dass** man bei der Bestimmung eine Immunreaktivität bestimmt, die den Positionen 147 bis 187 von LASP-1 (SEQ ID NO:1) zugeordnet werden kann.

4. Verfahren nach einem der Ansprüche 1 bis 3, **dadurch gekennzeichnet, dass** man die Immunreaktivität einer posttranslational gebildeten löslichen Form von LASP-1 und/oder LAP-1 bestimmt.

5. Verfahren nach einem der Ansprüche 1 bis 4, **dadurch gekennzeichnet, dass** es ein immundiagnostisches Bestimmungsverfahren (Immunoassay) vom Sandwichtyp ist.

6. Verfahren nach einem der Ansprüche 1 bis 5, **dadurch gekennzeichnet, dass** es ein immundiagnostisches Bestimmungsverfahren unter Einsatz von Antikörper-Reagenzien ist, die durch Immunisierung mit einem an ein Trägerprotein konjugierten Peptid erzeugt wurden, das ausgewählt ist aus Peptiden mit den Aminosäuresequenzen gemäß SEQ ID NO:13, SEQ ID NO:14 oder SEQ ID NO:17.

7. Verfahren nach einem der Ansprüche 1 bis 6, **dadurch gekennzeichnet, dass** es ein Verfahren zur Sepsisdiagnose ist und im Rahmen einer Multiparameter-Bestimmung neben der LASP-1-Immunreaktivität wenigstens ein weiterer Parameter bestimmt wird, der ausgewählt ist aus der Gruppe, die besteht aus Procalcitonin, CA 125, CA 19-9, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen, der Glycin-N-Acyltransferase (GNAT), der Carbamoylphosphat Synthetase 1 (CPS 1) und dem C-reaktiven Protein (CRP) oder Fragmenten davon.

8. Verfahren nach Anspruch 7, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

9. Verfahren nach Anspruch 8, **dadurch gekennzeichnet, dass** die Auswertung des mit der Messvorrichtung gewonnenen komplexen Messergebnisses mit Hilfe eines Computerprogramms erfolgt.

10. Verfahren nach einem der vorausgehenden Ansprüche, **dadurch gekennzeichnet, dass** man eine Bestimmung von LASP-1 und LAP-1 so durchführt, dass man beide gemeinsam bestimmt.

11. Verfahren nach einem der vorausgehenden Ansprüche, **dadurch gekennzeichnet, daß** man die Bestimmung von LASP-1 und/oder LAP-1 so durchführt, dass man Meßwerte gewinnt, die für die Anwesenheit und/oder Mengen nur von LASP-1 oder nur von LAP-1 kennzeichnend sind.

## Claims

1. Diagnostic in vitro method for early detection and detection, for prognosis of the course and for assessment of the severity and for therapy-accompanying monitoring of sepsis-like systemic infections and sepsis, cardiac infarction and Alzheimer's disease, in which in a serum or plasma sample of a patient the presence and/or amount of the protein LASP-1 (SEQ ID NO:1), or of a protein which is identical in sequence to it at least in the range of the first 200 amino acids and which has an immunoreactivity corresponding to said range, is determined in free and/or protein bound form, or in a posttranslationally modified form, and conclusions are drawn with respect to the presence, the expected course, the severity or the success of a therapy of the sepsis-like systemic infection or sepsis, the cardiac infarction or the Alzheimer's disease from the presence and/or amount of the LASP-1 immunoreactivity determined.

2. Method according to Claim 1, **characterized in that** in the determination the immunoreactivity of a protein or of a fragment thereof is determined or codetermined which is referred to as LAP-1 and has the amino acid sequence according to SEQ ID NO:16.

3. Method according to any of Claims 1 or 2, **characterized in that** in the determination an immunoreactivity is determined which can be assigned to positions 147 to 187 of LASP-1 (SEQ ID NO:1).

4. Method according to any of Claims 1 to 3, **characterized in that** the immunoreactivity of a posttranslationally formed soluble form of LASP-1 and/or LAP-1 is determined.

5. Method according to any of Claims 1 to 4, **characterized in that** it is an immunodiagnostic assay method (immunoassay) of the sandwich type.

6. Method according to any of Claims 1 to 5, **characterized in that** it is an immunodiagnostic assay method employing antibody reagents which were produced by immunization with a peptide which is conjugated with a carrier protein and which is selected from peptides having the amino acid sequences according to SEQ ID NO:13, SEQ ID NO:14 or SEQ ID NO:17.

7. Method according to any of Claims 1 to 6, **characterized in that** it is a method for sepsis diagnosis and, as part of a multiparameter determination, in addition to LASP-1 immunoreactivity at least one further parameter is determined which is selected from the group consisting of procalcitonin, CA 125, CA 19-9, S100B, S100A proteins, soluble cytokeratin fragments, in particular CYFRA 21, TPS and/or soluble cytokeratin-1 fragments (sCY1F), the peptides inflammin and CHP, peptide prohormones, glycine N-acyltransferase (GNAT), carbamoyl phosphate synthetase 1 (CPS 1) and the C-reactive protein (CRP), or fragments thereof.

8. Method according to Claim 7, **characterized in that** the multiparameter determination is carried out as a simultaneous determination by means of a chip technology measuring device or of an immunochromatographic measuring device.

9. Method according to Claim 8, **characterized in that** the evaluation of the complex measured result obtained using the measuring device is effected with the aid of a computer program.

10. Method according to any of the preceding Claims, **characterized in that** a determination of LASP-1 and LAP-1 is carried out in such a way that both are determined together.

11. Method according to any of the preceding Claims, **characterized in that** the determination of LASP-1 and/or LAP-1 is carried out in such a way that measured values which are characteristic of the presence and/or amounts of only LASP-1 and/or of only LAP-1 are obtained.

## Revendications

1. Procédé diagnostique in vitro pour la détection précoce et l'identification, pour le pronostic de l'évolution et l'évaluation du degré de gravité et pour l'évaluation de l'évolution concomitante à la thérapie d'infections systémiques, analogues à la septicémie et de la septicémie, de l'infarctus du myocarde et de la maladie d'Alzheimer, procédé dans lequel on met en évidence, dans un échantillon de sérum ou de plasma issu d'un patient, la présence et / ou la quantité de protéine LASP-1 (SEQ ID No. : 1) ou d'une protéine de séquence identique à celle-ci, au moins dans le domaine des 200 premiers acides aminés, et une immunoréactivité, qui correspond au domaine cité, de forme libre et / ou liée à la protéine ou modifiée au niveau de la posttranslation, et l'on tire, en fonction de la présence et / ou de la quantité de la réactivité immunologique LASP-1, des conclusions en ce qui concerne la présence, l'évolution attendue, le degré de gravité ou le succès de la thérapie de l'infection systémique, analogue à une septicémie, ou de la septicémie, de l'infarctus du myocarde ou de la maladie d'Alzheimer.

2. Procédé selon la revendication 1, **caractérisé en ce que**, lors de la détermination, on détermine la réactivité immunologique d'une protéine ou d'un fragment de celle-ci, que l'on désigne par LAP-1 et qui présente la séquence d'acide aminé selon SEQ ID No. : 16.

3. Procédé selon revendication 1 ou 2, **caractérisé en ce que**, lors de la détermination, on détermine une réactivité immunologique, qui peut être associée aux positions 147 à 187 de la LASP-1 (SEQ ID NO : 1).

4. Procédé selon l'une des revendications 1 à 3, **caractérisé en ce que** l'on détermine la réactivité immunologique d'une forme de LASP-1 et / ou de LAP-1 soluble, formée au niveau de la posttranslation.

5. Procédé selon l'une des revendications 1 à 4, **caractérisé en ce que** celui-ci est un procédé de détermination immunodiagnostique (essai immunologique) du type sandwich.

6. Procédé selon l'une des revendications 1 à 5, **caractérisé en ce que** celui-ci est un procédé de détermination immunodiagnostique, qui utilise des réactifs anticorps, qui ont été générés par immunisation avec un peptide, qui, par conjugaison avec une protéine de transport, est sélectionné dans le groupe des peptides avec séquence d'acides aminés selon SEQ ID No. : 13, SEQ ID No. : 14 ou SEQ ID No. : 17.

7. Procédé selon l'une des revendications 1 à 6, **caractérisé en ce que**, lors de la détermination multiparamètre, on détermine, à côté de la réactivité immunologique LASP-1, au moins un autre paramètre, qui est sélectionné dans le groupe qui comprend la procalcitonine, CA 125, CA 19-9, les protéines S100B, S100A, des fragments de cytokératine solubles, en particulier CYFRA 21, TPS, et / ou des fragments de cytokératine (sCY1F) solubles, des peptides inflammine et CHP, des pro-hormones peptidiques, la transférase de glycine N- Acyle (GNAT), la synthétase de phosphate carbamoylique 1 (CPS 1) et la protéine C réactive (CRP), ou des fragments de celles-ci.

8. Procédé selon la revendication 7, **caractérisé en ce que** la détermination multiparamètre est effectuée, en tant que détermination simultanée, au moyen d'un dispositif de mesure selon la technologie des microplaquettes ou d'un dispositif de mesure immunochromatographique.

9. Procédé selon la revendication 8, **caractérisé en ce que** l'évaluation des résultats de mesure, obtenus avec le dispositif de mesure, est effectuée à l'aide d'un programme d'ordinateur.

10. Procédé selon l'une des revendications précédentes, **caractérisé en ce que** l'on effectue une détermination de LASP-1 et de LAP-1 de sorte que toutes les deux soient déterminées en commun.

11. Procédé selon l'une des revendications précédentes, **caractérisé en ce que** l'on procède à la détermination de LASP-1 et de LAP-1 de sorte à obtenir des valeurs de mesure, qui sont caractéristiques seulement pour la présence et ou les quantités de LASP-1 et ou seulement pour celles de LAP-1.
